# EUROPEAN PATENT SPECIFICATION

(11) **EP 4 164 500 B1**
(45) Date of publication and mention of the grant of the patent: **14.02.2024**
(21) Application number: 22725911.6
(22) Date of filing: 05.05.2022
(51) Int. Cl.: A61B 6/00, A61B 6/10

(54) **MEDICAL IMAGING SYSTEM**
SYSTEM ZUR MEDIZINISCHEN BILDGEBUNG
SYSTÈME D'IMAGERIE MÉDICALE

(30) Priority: 11.05.2021 EP 21173268
(43) Date of publication of application: 19.04.2023
(73) Proprietor: Koninklijke Philips N.V., 5656 AG Eindhoven (NL)
(72) Inventor: LEUSSLER, Christoph Günther, 5656 AG Eindhoven (NL); FORTHMANN, Peter, 5656 AG Eindhoven (NL)
(74) Representative: Philips Intellectual Property & Standards
(86) International application number: PCT/EP2022/062119
(87) International publication number: WO 2022/238224

(56) References cited:
- CN-A- 111 436 964
- US-A1- 2020 205 753

## Description

### FIELD OF THE INVENTION

The present invention relates to a medical imaging system, a medical imaging method, as well as to a computer program element and a computer readable medium.

### BACKGROUND OF THE INVENTION

X-ray imaging systems utilize an X-ray tube to generate X-rays to irradiate specific parts of a patient, to obtain for example an X-ray attenuation image. To minimise exposure, metal shields are placed to block the X-rays from exposing irradiating parts of the patient not needing to be investigated. Hospitals and medical centres require a very high throughput of patients undergoing such examination. However, it can take a long time to position the shielding even with experienced operators, a problem that becomes exacerbated when less skilled operators are involved.

There is a need to resolve this issue CN111436964A discloses the use of a shield to protect the operator of a CT scanner, and mentions the use of a radar range finder.

### SUMMARY OF THE INVENTION

It would be advantageous to have an improved means of positioning patient shielding for an X-ray medical examination. The object of the present invention is solved with the subject matter of the independent claims, wherein further embodiments are incorporated in the dependent claims. It should be noted that the following described aspects and examples of the invention apply to the medical imaging system, and also to the medical imaging method as well as to the computer program element and the computer readable medium.

In a first aspect, there is provided medical imaging system, comprising:
- an X-ray image acquisition unit;
- a shield placement unit;
- a radar unit; and
- a processing unit.

The X-ray image acquisition unit is configured to acquire an X-ray image of a patient. The shield placement unit is configured to move at least one shield to cover at least one part of the patient to stop or limit X-ray exposure of the at least one part of the patient. The processing unit is configured to control the shield placement unit to move and position the at least one shield. The radar unit is configured to obtain radio frequency "RF" data. The RF data is obtained from the emission of RF radiation and the sensing of reflected RF radiation. The reflected RF radiation comprises RF radiation reflected from a shield of the at least one shield. The processing unit is configured to control the shield placement unit to move the shield with respect to the patient, and the control comprises utilization of the RF data.

Thus, automatic positioning and placement of radiation shielding is provided, providing for increased operational utilization, effectiveness and safety of x-ray imaging examinations.

In an example, the processing unit is configured to determine a location of the shield. The determination comprises utilization of the RF data, and the control of the shield placement unit can then comprise utilization of the location of the shield.

In an example, determination of the location of the shield comprises a determination of a location of an edge of the shield.

In other words, the Radar system is utilized to determine the location of the shield, because the return from a patient and the shield is different enabling the shield to be positioned as required.

In an example, the at least one shield comprises a plurality of shields. The processing unit is configured to determine an identity of the shield. The determination comprises utilization of the RF data reflected from the shield, and the control of the shield placement unit can then comprise utilization of the identity of the shield.

Thus, there can be a clinical situation, where there can be an X-Ray system and in effect a "therapy system" in combination or docked to each other. Then the shield is configured and monitored by the RF radiation. Thus, in the situation of a therapy application, the patient table can be moved and the radiation shield may need to have a different configuration. Different configuration means, that the shield does have a different absorption coefficient or surface structure to allow for optimal therapy, and by identifying the shield, it can be ensured that the correct shield and its correct placement is enabled by the medical imaging system.

In an example, determination of the identity of the shield comprises a determination of an RF signature associated with the shield.

In an example, each shield of the plurality of shields comprises a surface region with a different surface structure to surface structures of the other shields, and the RF signature of the shield can comprise a signature associated with the surface structure of the shield.

In an example, each shield of the plurality of shields comprises a material with a different absorption coefficient to absorption coefficients of material of the other shields, and the RF signature of the shield can comprise a signature associated with the material of the shield.

In an example, each shield of the plurality of shields has a size different to sizes of the other shields, and the RF signature of the shield can comprise a signature associated with the size of the shield.

Thus, the specific shield can be identified from the RF signature. This enables the correct shield to be positioned and also provides for a back up check to ensure that the correct shield has been moved by the shield placement unit.

In an example, the shield placement unit is configured to move the at least one shield horizontally in orthogonal directions.

In an example, the processing unit is configured to determine a size and/or location of the patient, the determination comprising utilization of the RF data. Control of the shield placement unit can then comprise utilization of the size and/or location of the patient determined from the RF data.

In an example, the medical imaging system comprises an infrared and/or visible/ or other electromagnetic radiation 3D imaging system configured to acquire 3D data. The processing unit is configured to determine a size and/or location of the patient, the determination comprising utilization of the 3D data. Control of the shield placement unit can then comprise utilization of the size and/or location of the patient determined from the 3D data.

In an example, the processing unit is configured to start and/or stop the X-ray image acquisition unit from emitting X-rays comprising utilization of the 3D data.

In an example, the processing unit is configured to start and/or stop the X-ray image acquisition unit from emitting X-rays comprising utilization of the RF data

In this manner, the system can only acquire X-ray data when it is determined that the shield or shields are in the correct position, and this can also be done based on the shields being correctly placed with respect to the patient. Also, if the patient moves, and a part of their body becomes uncovered, or an incorrect part of their body is being exposed with X-ray the system can determine this movement of the patient and stop the x-rays.

In a second aspect, there is provided a medical imaging method, comprising:
a) acquiring by an X-ray image acquisition unit an X-ray image of a patient;
b) moving by a shield placement unit at least one shield to cover at least one part of the patient to stop or limit X-ray exposure of the at least one part of the patient, wherein a processing unit is configured to control the shield placement unit to move and position the at least one shield;
c) obtaining by a radar unit radio frequency "RF" data, wherein the RF data is obtained from the emission of RF radiation and the sensing of reflected RF radiation, and wherein the reflected RF radiation comprises RF radiation reflected from a shield of the at least one shield; and
d) controlling by the processing unit the shield placement unit to move the shield with respect to the patient, and wherein the control comprises utilization of the RF data.

According to another aspect, there is provided a computer program element controlling one or more of the systems as previously described which, if the computer program element is executed by a processing unit, is adapted to perform the method as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic set up of an example of a medical imaging system;
Fig. 2 shows a medical imaging method;
Fig. 3 shows an example of a medical imaging system; and
Fig. 4 shows an example of a medical imaging system.

### DETAILED DESCRIPTION OF EMBODIMENTS

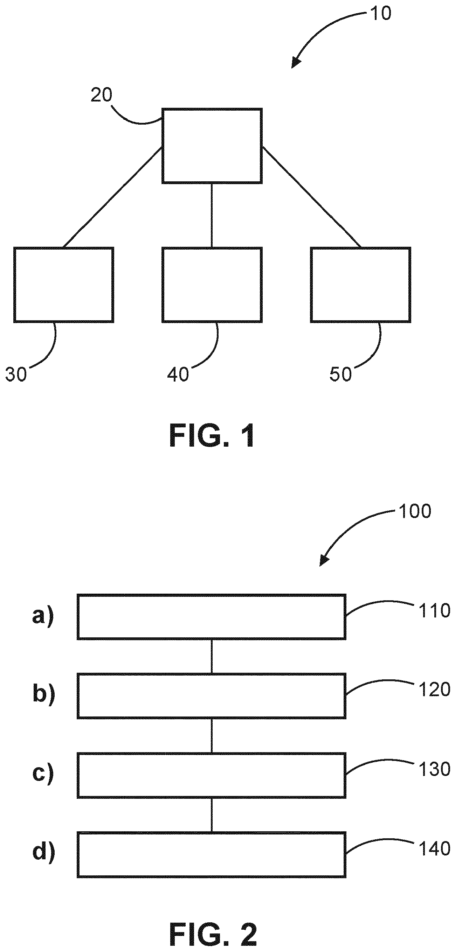

Fig. 1 shows a schematic example of a medical imaging system 10. The system comprises an X-ray image acquisition unit 20, a shield placement unit 30, a radar unit 40, and a processing unit 50. The X-ray image acquisition unit is configured to acquire an X-ray image of a patient. The shield placement unit is configured to move at least one shield to cover at least one part of the patient to stop or limit X-ray exposure of the at least one part of the patient. The processing unit is configured to control the shield placement unit to move and position the at least one shield. The radar unit is configured to obtain radio frequency "RF" data. The RF data is obtained from the emission of RF radiation and the sensing of reflected RF radiation, and the reflected RF radiation comprises RF radiation reflected from a shield of the at least one shield. The processing unit is configured to control the shield placement unit to move the shield with respect to the patient, and the control comprises utilization of the RF data.

In an example, the at least one shield comprises a plurality of shields, and the reflected RF radiation comprises RF radiation reflected from each shield of the plurality of shields. The processing unit is configured to move each shield of the plurality of shields with respect to the patient, and the control comprises utilization of the RF data.

In an example, the processing unit is configured to determine a location of each shield of the plurality of shields, and the determination comprises utilization of the RF data. The control of the shield placement unit can comprise utilization of the location of each shield.

In an example, determination of the location of each shield comprises a determination of a location of an edge of each shield.

In an example, each shield comprises metal.

According to an example, the processing unit is configured to determine a location of the shield that the shield placement unit is to move or moving, and the determination comprises utilization of the RF data. The control of the shield placement unit can comprise utilization of the location of the shield.

According to an example, determination of the location of the shield comprises a determination of a location of an edge of the shield.

According to an example, the at least one shield comprises a plurality of shields. The processing unit is configured to determine an identity of the shield that the shield placement unit is to move or moving. The determination comprises utilization of the RF data reflected from the shield, and the control of the shield placement unit can comprise utilization of the identity of the shield.

According to an example, determination of the identity of the shield comprises a determination of an RF signature associated with the shield.

According to an example, each shield of the plurality of shields comprises a surface region with a different surface structure to surface structures of the other shields. The RF signature of the shield that the shield placement unit is to move or moving can comprise a signature associated with the surface structure of the shield.

According to an example, each shield of the plurality of shields comprises a material with a different absorption coefficient to absorption coefficients of material of the other shields. The RF signature of the shield that the shield placement unit is to move or moving can comprise a signature associated with the material of the shield.

According to an example, each shield of the plurality of shields has a size different to sizes of the other shields. The RF signature of the shield that the shield placement unit is to move or moving can comprise a signature associated with the size of the shield.

In an example, the processing unit utilizes a database of RF signatures to identify the shield.

Thus, shields with different sizes and/or with different surface textures or surfaces and/or with different absorption coefficients exhibit different radar signatures. An acquired radar signature for a shield being moved can then be compared with the database to conform the identity of the shield.

According to an example, the shield placement unit is configured to move the at least one shield horizontally in orthogonal directions.

In an example, the shield placement unit is configured to move the at least one shield vertically.

According to an example, the processing unit is configured to determine a size and/or location of the patient, the determination comprising utilization of the RF data. Control of the shield placement unit can comprise utilization of the size and/or location of the patient determined from the RF data.

According to an example, the medical imaging system comprises an infrared and/or visible/or other electromagnetic radiation 3D imaging system configured to acquire 3D data. The processing unit is configured to determine a size and/or location of the patient, the determination comprising utilization of the 3D data. Control of the shield placement unit can comprise utilization of the size and/or location of the patient determined from the 3D data.

Thus, visible, infrared can be utilized. Also, radiation in the range of 200-300 GHz, for example, that can be utilized that enables 3D information to be provided with a mm resolution, which can still be sufficient for shielded monitoring.

According to an example, the processing unit is configured to start and/or stop the X-ray image acquisition unit from emitting X-rays comprising utilization of the 3D data.

According to an example, the processing unit is configured to start and/or stop the X-ray image acquisition unit from emitting X-rays comprising utilization of the RF data

Fig. 2 shows an example of a medical imaging method 100 in its basic steps. The method comprises:
- in an acquiring step 110, also referred to as step a), acquiring by an X-ray image acquisition unit an X-ray image of a patient;
- in a moving step 120, also referred to as step b) moving by a shield placement unit at least one shield to cover at least one part of the patient to stop or limit X-ray exposure of the at least one part of the patient, wherein a processing unit is configured to control the shield placement unit to move and position the at least one shield;
- in obtaining step 130, also referred to as step c) obtaining by a radar unit radio frequency "RF" data, wherein the RF data is obtained from the emission of RF radiation and the sensing of reflected RF radiation, and wherein the reflected RF radiation comprises RF radiation reflected from a shield of the at least one shield; and
- in a controlling step 140, also referred to as step d) controlling by the processing unit the shield placement unit to move the shield with respect to the patient, and wherein the control comprises utilization of the RF data.

In an example, the at least one shield comprises a plurality of shields, and the reflected RF radiation comprises RF radiation reflected from each shield of the plurality of shields. The processing unit is configured to control the shield placement unit to move each shield of the plurality of shields with respect to the patient, and step d) can comprise utilization of the RF data.

In an example, the processing unit is configured to determine a location of each shield of the plurality of shields, wherein the determination comprises utilization of the RF data, and step d) can comprise utilization of the location of each shield.

In an example, determination of the location of each shield comprises a determination of a location of an edge of each shield.

In an example, the method comprises determining by the processing unit a location of the shield that the shield placement unit is to move or moving, and the determining comprises utilizing the RF data, and step d) can comprise utilizing the location of the shield.

In an example, determining the location of the shield comprises determining a location of an edge of the shield.

In an example, the at least one shield comprises a plurality of shields, and method the comprises determining by the processing an identity of the shield, and the determining comprises utilizing the RF data reflected from the shield, and step d) can comprise utilizing the identity of the shield.

In an example, determining the identity of the shield comprises determining an RF signature associated with the shield.

In an example, each shield of the plurality of shields comprises a surface region with a different surface structure to surface structures of the other shields, and the RF signature of the shield that the shield placement unit is to move or moving comprises a signature associated with the surface structure of the shield.

In an example, each shield of the plurality of shields comprises a material with a different absorption coefficient to absorption coefficients of material of the other shields, and the RF signature of the shield that the shield placement unit is to move or moving comprises a signature associated with the material of the shield.

In an example, each shield of the plurality of shields has a size different to sizes of the other shields, and the RF signature of the shield that the shield placement unit is to move or moving comprises a signature associated with the size of the shield.

In an example, step b) comprises moving by the shield placement unit the at least one shield horizontally in orthogonal directions.

In an example, step b) comprises moving by shield placement unit the at least one shield vertically.

In an example, the method comprises determining by the processing unit a size and/or location of the patient, the determining comprising utilizing the RF data, and step d) comprises utilizing the size and/or location of the patient determined from the RF data.

In an example, the method comprises utilizing an infrared/electromagnetic and/or visible/ or other electromagnetic radiation 3D imaging system to acquire 3D data, and the method comprises determining by the processing unit a size and/or location of the patient, the determining comprising utilizing the 3D data, and step d) comprises utilizing the size and/or location of the patient determined from the 3D data.

In an example, the method comprises starting and/or stopping by the processing unit the X-ray image acquisition unit from emitting X-rays comprising utilizing the 3D data.

In an example, the method comprises starting and/or stopping by the processing unit the X-ray image acquisition unit from emitting X-rays comprising utilizing the RF data.

Thus, radiation protection is used during X-ray imaging to protect parts of the body from being exposed to the X-ray radiation. To control the exact fitting and give guidance to how to position the shielding an RF sensor system is used, which detects the location of the shield such as a metallic device. In this manner, fast and accurate execution of the scan process can be enabled, which also allows the position of the patient to be taken into account, which enables for example a scan abort if the patient moves inappropriately. This can also be augmented via three dimensional imaging.

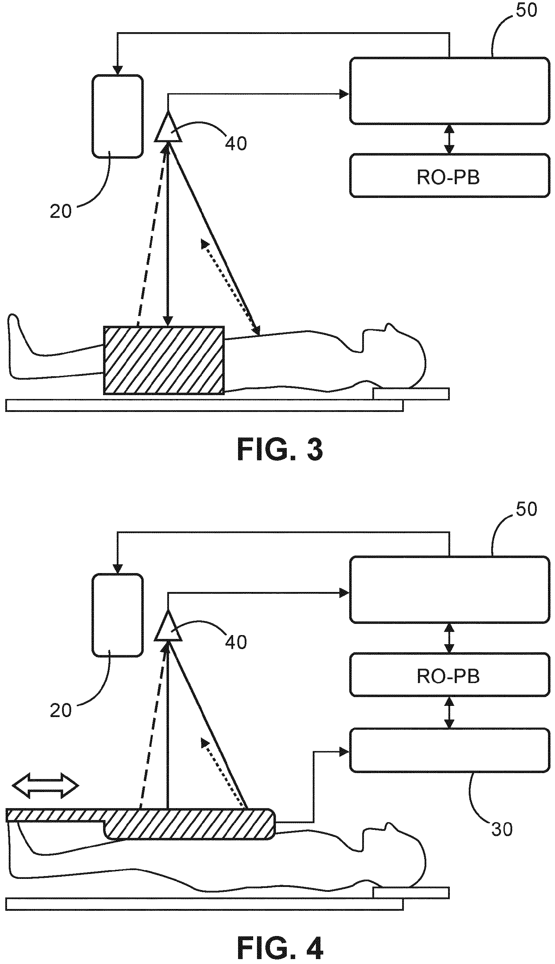

The medical imaging system and medical imaging method are further explained in specific detail, where reference is made to Figs. 3-4.

The new technique provides for the automatic detection and placement checking of radiation protection wearables for use in an (autonomous) X-ray unit/system. The X-Ray shielding can consist of a metallic radiation shield and can be exactly monitored using a RADAR system - other radiation shields can be utilized. The radar signals are reflected from the metallic surface, whereas the radiation, which hits different surfaces such as clothing or skin is absorbed/reflected differently, thus a contrast between the shield and the patient and/or areas is visible. A feedback loop controls the positioning during preparation and imaging. Thus, the patient and staff are guided for correct placement. The system can be completely autonomous, with a processing unit controlling shield placement without operator interaction, or an operator can be in the loop who actually provides input to the processing unit to move the shield and movement can be stopped when the shield has moved to the correct position.

As shown in Fig.3, a patient is to be scanned via an X-ray image acquisition unit 20 that utilizes an X-ray tube, and where a radiation shield has been placed to block X-rays. A radar unit 40 has a radio frequency emitter and an RF an electromagnetic sensor that detects the reflected RF beam from the metallic surface of the radiation shield. The solid lines from the radar unit 40 indicate RF radiation emitted by the emitter of the radar unit that interacts with the patient and with the radiation shield, with the long dashed line indicating RF radiation that is reflected by the radiation shield and sensed by the sensor of the radar unit with the short dashed line indicating RF radiation that is reflected by the patient and sensed by the sensor of the radar unit. The sensor is connected to a feedback control logic within a processing unit 50, which controls the switching of the X-Ray tube. Only if the protection is located in its correct position imaging is the scan started. If during imaging the shield changes its position due to motion, the scan sequence is stopped. The system can also provide interactive guidance for the patient during preparation. In Fig. 3, RO-PB indicates a link to a remote operator and database. It is to be noted that the "radar unit" can come in many forms, with one example being a radar system using a radar array to use beam focusing and direction to scan the surface of the patient and shield, thus can be for example a phased array system, with another example being a radar system connected to a mechanical unit (gimbal) to scan the surface of the patient/shield/surroundings.

The patient can place himself the radiation shield. With the feedback system, less qualified persons can locate the radiation shield. Exact positioning can be better controlled by a remote operator, who is located in a remote command operating centre. The radar beam detection provides a safe and very reliable detection method, even when the shield is hidden by clothes. The technique can be further combined by 3D sensing such as LIDAR/video. The 3 D sensing system detects size and location and posing of the patient and RADAR detects exact positioning of radiation shield with respect to patient. A software program on the processing unit calculates the correct positioning/placement of the shield for optimal X-Ray dose protection.

Fig. 4 shows a fully automated version of the system of Fig. 3, where the radiation shield/mask is integrated in the X-Ray diagnostic scanner and is placed automatically via a transport system or shield placement unit 10to the exact location. The system can automatically select between different shields of different absorption characteristics and sizes.

Thus, dependent upon the diagnostic problem being addressed, the size and location of the part of the patient to be imaged or the X-ray window are determined by the diagnostic problem. The radiation shields can be translated from left, right, front, back (4 directions). Individual shields can have a structured surface for the Radar signal to improve signal contrast and provide individual signatures for the shields, enabling the different shields to be identified. This can be detected by the RF structured surface. This enables the overlap region between the shield and the area outside the shield, such as the patient, to be clearly distinguished and to identify the shield (it is to be noted that individual shields can have different sizes and absorption coefficients). It is also to be noted that the patient can wear garments with a specific structure, helping the patient to be identified and can be equipped and positioned with portable or wearable shielding garments.

The radar system can be utilized itself to provide 3D information, where a broadband pulse radar is utilized for example to provide 3D information. Further information can be found at: **https://phys.org/news/2017-10-d-rooms-radar.html**. The sensing of 3D RADAR signals can provides information for the detection and correct location of the X-Ray radiation shields, which can involves utilization of a trained neural network. However such 3D data is not necessary as such.

The overall system can utilize a 3D camera/lidar, which can be used to obtain 3D image data to accurately locate the patient. This visible/infrared/mm wavelength 3D data can be used separately to the radar data, or in effect fused with the radar data in a similar fashion to advanced driving light/radar systems.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this patent.

However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A medical imaging system (10), comprising:
- an X-ray image acquisition unit (20);
- a shield placement unit (30);
- a radar unit (40); and
- a processing unit (50);
wherein the X-ray image acquisition unit is configured to acquire an X-ray image of a patient;
wherein the shield placement unit is configured to move at least one shield to cover at least one part of the patient to stop or limit X-ray exposure of the at least one part of the patient;
wherein the processing unit is configured to control the shield placement unit to move and position the at least one shield;
wherein the radar unit is configured to obtain radio frequency "RF" data, wherein the RF data is obtained from the emission of RF radiation and the sensing of reflected RF radiation, and wherein the reflected RF radiation comprises RF radiation reflected from a shield of the at least one shield; and
wherein the processing unit is configured to control the shield placement unit to move the shield with respect to the patient, and wherein the control comprises utilization of the RF data.

2. Medical imaging system according to claim 1, wherein the processing unit is configured to determine a location of the shield, wherein the determination comprises utilization of the RF data, and wherein the control of the shield placement unit comprises utilization of the location of the shield.

3. Medical imaging system according to claim 2, wherein determination of the location of the shield comprises a determination of a location of an edge of the shield.

4. Medical imaging system according to any of claims 1-3, wherein the at least one shield comprises a plurality of shields, wherein the processing unit is configured to determine an identity of the shield, wherein the determination comprises utilization of the RF data reflected from the shield, and wherein the control of the shield placement unit comprises utilization of the identify of the shield.

5. Medical imaging system according to claim 4, wherein determination of the identity of the shield comprises a determination of an RF signature associated with the shield.

6. Medical imaging system according to any of claims 4-5, wherein each shield of the plurality of shields comprises a surface region with a different surface structure to surface structures of the other shields, and wherein the RF signature of the shield comprises a signature associated with the surface structure of the shield.

7. Medical imaging system according to any of claims 4-6, wherein each shield of the plurality of shields comprises a material with a different absorption coefficient to absorption coefficients of material of the other shields, and wherein the RF signature of the shiled comprises a signature associated with the material of the shield.

8. Medical imaging system according to any of claims 4-7, wherein each shield of the plurality of shields has a size different to sizes of the other shields, and wherein the RF signature of the shiled comprises a signature associated with the size of the shield.

9. Medical imaging system according to any of claims 1-8, wherein the shield placement unit is configured to move the at least one shield horizontally in orthogonal directions.

10. Medical imaging system according to any of claims 1-9, wherein the processing unit is configured to determine a size and/or location of the patient, the determination comprising utilization of the RF data, and wherein control of the shield placement unit comprises utilization of the size and/or location of the patient determined from the RF data.

11. Medical imaging system according to any of claims 1-10, wherein the medical imaging system comprises an infrared and/or visible and/or other electromagnetic radiation 3D imaging system configured to acquire 3D data, and wherein the processing unit is configured to determine a size and/or location of the patient, the determination comprising utilization of the 3D data, and wherein control of the shield placement unit comprises utilization of the size and/or location of the patient determined from the 3D data.

12. Medical imaging system according to claim 11, wherein the processing unit is configured to start and/or stop the X-ray image acquisition unit from emitting X-rays comprising utilization of the 3D data.

13. Medical imaging system according to any of claims 1-12, wherein the processing unit is configured to start and/or stop the X-ray image acquisition unit from emitting X-rays comprising utilization of the RF data

14. A medical imaging method (100), comprising:
a) acquiring (110) by an X-ray image acquisition unit an X-ray image of a patient;
b) moving (120) by a shield placement unit at least one shield to cover at least one part of the patient to stop or limit X-ray exposure of the at least one part of the patient, wherein a processing unit is configured to control the shield placement unit to move and position the at least one shield;
c) obtaining (130) by a radar unit radio frequency "RF" data, wherein the RF data is obtained from the emission of RF radiation and the sensing of reflected RF radiation, and wherein the reflected RF radiation comprises RF radiation reflected from a shield of the at least one shield; and
d) controlling (140) by the processing unit the shield placement unit to move the shield with respect to the patient, and wherein the control comprises utilization of the RF data.

15. A computer program element for controlling a system according to any one of claims 1 to 13, which when executed by a processor is configured to carry out the method of claim 14.

## Patentansprüche

1. Medizinisches Bildgebungssystem (10), umfassend:
- eine Röntgenbilderfassungseinheit (20);
- eine Schildplatzierungseinheit (30);
- eine Radareinheit (40); und
- eine Verarbeitungseinheit (50); wobei die Röntgenbilderfassungseinheit dazu konfiguriert ist, ein Röntgenbild eines Patienten zu erfassen; wobei die Schutzschildplatzierungseinheit so konfiguriert ist, dass sie mindestens einen Schutzschild bewegt, um mindestens einen Teil des Patienten abzudecken, um die Röntgenstrahlungsbelastung des mindestens eines Teils des Patienten zu stoppen oder zu begrenzen; wobei die Verarbeitungseinheit so konfiguriert ist, dass sie die Schildplatzierungseinheit steuert, um den mindestens einen Schild zu bewegen und zu positionieren; wobei die Radareinheit so konfiguriert ist, dass sie Hochfrequenz-"RF"-Daten erhält, wobei die RF-Daten aus der Emission von RF-Strahlung und der Erfassung reflektierter RF-Strahlung gewonnen werden und wobei die reflektierte RF-Strahlung von einer Abschirmung reflektierte RF-Strahlung umfasst mindestens ein Schild; und wobei die Verarbeitungseinheit so konfiguriert ist, dass sie die Schutzschildplatzierungseinheit steuert, um den Schutzschild in Bezug auf den Patienten zu bewegen, und wobei die Steuerung die Nutzung der RF-Daten umfasst.

2. Medizinisches Bildgebungssystem nach Anspruch 1, wobei die Verarbeitungseinheit dazu konfiguriert ist, einen Ort des Schildes zu bestimmen, wobei die Bestimmung die Nutzung der RF-Daten umfasst und wobei die Steuerung der Schildplatzierungseinheit die Nutzung des Ortes des Schildes umfasst.

3. Medizinisches Bildgebungssystem nach Anspruch 2, wobei die Bestimmung der Position des Schildes eine Bestimmung einer Position einer Kante des Schildes umfasst.

4. Medizinisches Bildgebungssystem nach einem der Ansprüche 1-3, wobei die mindestens eine Abschirmung mehrere Abschirmungen umfasst, wobei die Verarbeitungseinheit dazu konfiguriert ist, eine Identität der Abschirmung zu bestimmen, wobei die Bestimmung die Nutzung der von der Abschirmung reflektierten RF-Daten umfasst Schild, und wobei die Steuerung der Schildplatzierungseinheit die Verwendung der Identität des Schildes umfasst.

5. Medizinisches Bildgebungssystem nach Anspruch 4, wobei die Bestimmung der Identität des Schildes eine Bestimmung einer mit dem Schild verbundenen RF-Signatur umfasst.

6. Medizinisches Bildgebungssystem nach einem der Ansprüche 4-5, wobei jede Abschirmung der Vielzahl von Abschirmungen einen Oberflächenbereich mit einer Oberflächenstruktur aufweist, die sich von den Oberflächenstrukturen der anderen Abschirmungen unterscheidet, und wobei die RF-Signatur der Abschirmung eine damit verbundene Signatur umfasst die Oberflächenstruktur des Schildes.

7. Medizinisches Bildgebungssystem nach einem der Ansprüche 4-6, wobei jede Abschirmung der mehreren Abschirmungen ein Material mit einem anderen Absorptionskoeffizienten als die Absorptionskoeffizienten des Materials der anderen Abschirmungen umfasst und wobei die RF-Signatur der Abschirmung eine zugehörige Signatur umfasst mit dem Material des Schildes.

8. Medizinisches Bildgebungssystem nach einem der Ansprüche 4-7, wobei jede Abschirmung der mehreren Abschirmungen eine Größe aufweist, die sich von den Größen der anderen Abschirmungen unterscheidet, und wobei die RF-Signatur der Abschirmung eine Signatur umfasst, die der Größe der Abschirmung zugeordnet ist.

9. Medizinisches Bildgebungssystem nach einem der Ansprüche 1-8, wobei die Schildplatzierungseinheit so konfiguriert ist, dass sie den mindestens einen Schild horizontal in orthogonalen Richtungen bewegt.

10. Medizinisches Bildgebungssystem nach einem der Ansprüche 1-9, wobei die Verarbeitungseinheit dazu konfiguriert ist, eine Größe und/oder Lage des Patienten zu bestimmen, wobei die Bestimmung die Nutzung der RF-Daten umfasst, und wobei die Steuerung der Schildplatzierungseinheit die Nutzung von umfasst die aus den RF-Daten ermittelte Größe und/oder Lage des Patienten.

11. Medizinisches Bildgebungssystem nach einem der Ansprüche 1-10, wobei das medizinische Bildgebungssystem ein 3D-Bildgebungssystem mit Infrarot- und/oder sichtbarer und/oder anderer elektromagnetischer Strahlung umfasst, das zum Erfassen von 3D-Daten konfiguriert ist, und wobei die Verarbeitungseinheit zum Bestimmen einer Größe konfiguriert ist und/oder Lage des Patienten, wobei die Bestimmung die Nutzung der 3D-Daten umfasst, und wobei die Steuerung der Schildplatzierungseinheit die Nutzung der Größe und/oder Lage des Patienten umfasst, die aus den 3D-Daten bestimmt werden.

12. Medizinisches Bildgebungssystem nach Anspruch 11, wobei die Verarbeitungseinheit so konfiguriert ist, dass sie die Röntgenbilderfassungseinheit unter Verwendung der 3D-Daten startet und/oder stoppt, damit sie Röntgenstrahlen aussendet.

13. Medizinisches Bildgebungssystem nach einem der Ansprüche 1-12, wobei die Verarbeitungseinheit so konfiguriert ist, dass sie die Röntgenbilderfassungseinheit unter Verwendung der RF-Daten startet und/oder stoppt, damit sie Röntgenstrahlen aussendet.

14. Medizinisches Bildgebungsverfahren (100), umfassend:
a) erfassen (110) eines Röntgenbildes eines Patienten durch eine Röntgenbilderfassungseinheit;
b) bewegen (120) mindestens eines Schutzschilds durch eine Schutzschildplatzierungseinheit, um mindestens einen Teil des Patienten abzudecken, um die Röntgenstrahlungsexposition des mindestens eines Teils des Patienten zu stoppen oder zu begrenzen, wobei eine Verarbeitungseinheit zum Steuern des Schutzschilds konfiguriert ist Platzierungseinheit zum Bewegen und Positionieren des mindestens eines Schildes;
c) erhalten (130) von Hochfrequenz-"RF"-Daten durch eine Radareinheit, wobei die RF-Daten aus der Emission von RF-Strahlung und der Erfassung reflektierter RF-Strahlung erhalten werden und wobei die reflektierte RF-Strahlung von einer Abschirmung der Radareinheit reflektierte RF-Strahlung umfasst mindestens ein Schild; und
d) Steuern (140) der Schildplatzierungseinheit durch die Verarbeitungseinheit, um den Schild in Bezug auf den Patienten zu bewegen, und wobei die Steuerung die Nutzung der RF-Daten umfasst.

15. Computerprogrammelement zur Steuerung eines Systems nach einem der Ansprüche 1 bis 13, das bei Ausführung durch einen Prozessor dazu konfiguriert ist, das Verfahren nach Anspruch 14 auszuführen.

## Revendications

1. Système d'imagerie médicale (10) comprenant:
- une unité d'acquisition d'images radiographiques (20) ;
- une unité de positionnement de bouclier (30);
- une unité radar (40); et
- une unité de traitement (50); dans lequel l'unité d'acquisition d'images radiographiques est configurée pour acquérir une image radiographique d'un patient; dans lequel l'unité de positionnement de bouclier est configurée pour déplacer au moins un bouclier afin de couvrir au moins une partie du patient pour arrêter ou limiter l'exposition aux rayons X d'au moins une partie du patient; dans lequel l'unité de traitement est configurée pour commander l'unité de positionnement de bouclier afin de déplacer et de positionner le bouclier au moins; dans lequel l'unité radar est configurée pour obtenir des données de radiofréquence "RF", les données RF étant obtenues par l'émission d'un rayonnement de radiofréquence et la détection d'un rayonnement de radiofréquence réfléchi, et dans lequel le rayonnement RF réfléchi comprend le rayonnement RF réfléchi par un bouclier de l'au moins un bouclier; et dans lequel l'unité de traitement est configurée pour commander l'unité de positionnement de bouclier afin de déplacer le bouclier par rapport au patient, et dans lequel la commande comprend l'utilisation des données RF.

2. Système d'imagerie médicale selon la revendication 1, dans lequel l'unité de traitement est configurée pour déterminer l'emplacement du bouclier, dans lequel la détermination comprend l'utilisation des données RF, et dans lequel la commande de l'unité de positionnement de bouclier comprend l'utilisation de l'emplacement du bouclier.

3. Système d'imagerie médicale selon la revendication 2, dans lequel la détermination de l'emplacement du bouclier comprend la détermination de l'emplacement d'un bord du bouclier.

4. Système d'imagerie médicale selon l'une des revendications 1-3, dans lequel l'au moins un bouclier comprend plusieurs boucliers, dans lequel l'unité de traitement est configurée pour déterminer l'identité du bouclier, dans lequel la détermination comprend l'utilisation des données RF réfléchies par le bouclier, et dans lequel la commande de l'unité de positionnement du bouclier comprend l'utilisation de l'identification du bouclier.

5. Système d'imagerie médicale selon la revendication 4, dans lequel la détermination de l'identité du bouclier comprend la détermination d'une signature RF associée au bouclier.

6. Système d'imagerie médicale selon l'une des revendications 4-5, dans lequel chaque bouclier de la pluralité de boucliers comprend une région de surface avec une structure de surface différente des structures de surface des autres boucliers, et dans lequel la signature RF du bouclier comprend une signature associée à la structure de surface du bouclier.

7. Système d'imagerie médicale selon l'une des revendications 4-6, dans lequel chaque bouclier de la pluralité de boucliers comprend un matériau ayant un coefficient d'absorption différent des coefficients d'absorption des matériaux des autres boucliers, et dans lequel la signature RF du bouclier comprend une signature associée au matériau du bouclier.

8. Système d'imagerie médicale selon l'une des revendications 4-7, dans lequel chaque bouclier de la pluralité de boucliers a une taille différente de celle des autres boucliers, et dans lequel la signature RF du bouclier comprend une signature associée à la taille du bouclier.

9. Système d'imagerie médicale selon l'une des revendications 1-8, dans lequel l'unité de positionnement du bouclier est configurée pour déplacer le bouclier au moins horizontalement dans des directions orthogonales.

10. Système d'imagerie médicale selon l'une des revendications 1-9, dans lequel l'unité de traitement est configurée pour déterminer la taille et/ou l'emplacement du patient, la détermination comprenant l'utilisation des données RF, et dans lequel la commande de l'unité de positionnement du bouclier comprend l'utilisation de la taille et/ou de l'emplacement du patient déterminé à partir des données RF.

11. Système d'imagerie médicale selon l'une des revendications 1-10, dans lequel le système d'imagerie médicale comprend un système d'imagerie 3D infrarouge et/ou visible et/ou à autre rayonnement électromagnétique configuré pour acquérir des données 3D, et dans lequel l'unité de traitement est configurée pour déterminer une taille et/ou un emplacement du patient, la détermination comprenant l'utilisation des données 3D, et dans lequel la commande de l'unité de positionnement du bouclier comprend l'utilisation de la taille et/ou de l'emplacement du patient déterminé à partir des données 3D.

12. Système d'imagerie médicale selon la revendication 11, dans lequel l'unité de traitement est configurée pour démarrer et/ou arrêter l'unité d'acquisition d'images radiographiques d'émettre des rayons X en utilisant les données 3D.

13. Système d'imagerie médicale selon l'une des revendications 1-12, dans lequel l'unité de traitement est configurée pour démarrer et/ou arrêter l'unité d'acquisition d'images radiographiques d'émettre des rayons X en utilisant les données RF.

14. Méthode d'imagerie médicale (100) comprenant:
a) l'acquisition (110) par une unité d'acquisition d'images radiographiques d'une image radiographique d'un patient;
b) le déplacement (120) par une unité de positionnement de bouclier d'au moins un bouclier pour couvrir au moins une partie du patient afin d'arrêter ou de limiter l'exposition aux rayons X d'au moins une partie du patient, une unité de traitement étant configurée pour commander l'unité de positionnement de bouclier afin de déplacer et de positionner au moins un bouclier;
c) l'obtention (130) par une unité radar de données de radiofréquence "RF", les données RF étant obtenues par l'émission d'un rayonnement RF et la détection d'un rayonnement RF réfléchi, et le rayonnement RF réfléchi comprenant le rayonnement RF réfléchi par un bouclier de l'au moins un bouclier; et
d) la commande (140) par l'unité de traitement de l'unité de positionnement du bouclier pour déplacer le bouclier par rapport au patient, et la commande comprenant l'utilisation des données RF.

15. Élément de programme informatique pour commander un système selon l'une des revendications 1 à 13, qui, lorsqu'il est exécuté par un processeur, est configuré pour mettre en œuvre la méthode de la revendication 14.
